# EUROPEAN PATENT APPLICATION

(11) **EP 4 219 806 A2**
(43) Date of publication of application: **02.08.2023**
(21) Application number: 23157562.2
(22) Date of filing: 11.04.2018
(51) Int. Cl.: C40B 40/02, C12N 15/10, C12N 15/86, C12N 7/00

(54) **ADENO-ASSOCIATED VIRUS LIBRARY**

(30) Priority: 11.04.2017 EP 17165906
(62) Divisional of application: 18716286.2
(71) Applicant: Ruprecht-Karls-Universität Heidelberg, 69117 Heidelberg (DE)
(72) Inventor: Grimm, Dirk, 69117 Heidelberg (DE); Börner, Kathleen, 69198 Heidelberg (DE)
(74) Representative: Altmann Stößel Dick Patentanwälte PartG mbB

(57) **Abstract**

The present invention relates to an Adeno-associated virus (AAV) library comprising a multitude of Adeno-associated virus (AAV) variants, wherein the viral particles of each AAV variant comprise a specific combination of (i) a strain-specific capsid polypeptide and (ii) a peptide inserted into said capsid polypeptide, and wherein said strain-specific capsid polypeptides are derived from at least two different AAV strains. The present invention further relates to a method for identifying an AAV variant infecting a target cell of interest comprising a) providing a library of AAV variants comprising each AAV variant in an isolated manner, wherein each AAV variant of said library of AAV variants comprises a specific combination of (i) a strain-specific capsid polypeptide and (ii) a peptide inserted into said capsid polypeptide and wherein said strain-specific capsid polypeptides are derived from at least two different AAV strains; b) contacting each of said AAV variants individually with said target cell of interest; c) determining AAV infection of said target cells of interest; and d) based on the result of step c), identifying a variant AAV infecting a target cell of interest; and to a library of polynucleotides, a library of cloning cells, and to uses related thereto.

## Description

The present invention relates to an Adeno-associated virus (AAV) library comprising a multitude of Adeno-associated virus variants, wherein the viral particles of each AAV variant comprise a specific combination of (i) a strain-specific capsid polypeptide and (ii) a peptide inserted into said capsid polypeptide, and wherein said strain-specific capsid polypeptides are derived from at least two different AAV strains. The present invention further relates to a method for identifying an AAV variant infecting a target cell of interest comprising a) providing a library of AAV variants comprising each AAV variant in an isolated manner, wherein each AAV variant of said library of AAV variants comprises a specific combination of (i) a strain-specific capsid polypeptide and (ii) a peptide inserted into said capsid polypeptide and wherein said strain-specific capsid polypeptides are derived from at least two different AAV strains; b) contacting each of said AAV variants individually with said target cell of interest; c) determining AAV infection of said target cells of interest; and d) based on the result of step c), identifying a variant AAV infecting a target cell of interest; and to a library of polynucleotides, a library of cloning cells and to uses related thereto.

Adeno-associated virus (AAV) vectors have been known to enable transfer and long-term expression of foreign DNA including therapeutic genes in a variety of cell types. The different serotypes of AAV have been shown to have different tropisms in cultured cells and within the body, with AAV9 e.g. having the highest tropism for the murine heart. Transduction efficiencies and cell specificities obtainable with AAV vectors comprising unmodified capsids are good for some cell types, however, they are far too low to be of experimental or therapeutic use for most other cell types.

The feasibility of using random peptide display libraries to select for AAV vectors with improved efficiency and specificity for gene transfer into specific cell types has been shown. Such proceeding, however, has been found to be time-consuming and labor-intensive, since several rounds of enrichment, virus DNA rescue (e.g., by PCR or next generation sequencing) and reinfection are required to identify suitable binding peptides. Moreover, numerous examples in the literature show that peptides which have become most enriched after multiple selection rounds in a given target cell are not necessarily the best candidates with respect to efficiency and specificity, for instance Sallach et al. (2014), Mol Ther 22(5):929 and Michelfelder et al. (2007), Exp Hematol 35(12): 1766. Consequently, several of the enriched peptides are typically subcloned individually and then tested separately, which further increases the work load and time required to eventually identify desired candidates through this conventional approach.

There is, thus, a need in the art for improved means and methods for identifying AAV vectors with improved infection of selected target cells avoiding at least in part the drawbacks of the prior art. In particular, there is a need for novel strategies that substantially accelerate the screening process. This problem is solved by the means and methods of the present invention with the features of the independent claims. Preferred embodiments, which might be realized in an isolated fashion or in any arbitrary combination, are listed in the dependent claims.

Accordingly, the present invention relates to an Adeno-associated virus (AAV) library comprising a multitude of Adeno-associated virus variants, wherein the viral particles of each AAV variant comprise a specific combination of (i) a strain-specific capsid polypeptide and (ii) a peptide inserted into said capsid polypeptide, and wherein said strain-specific capsid polypeptides are derived from at least two different AAV strains.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention. Moreover, if not otherwise indicated, the term "about" relates to the indicated value with the commonly accepted technical precision in the relevant field, preferably relates to the indicated value ± 20%, more preferably ± 10%, most preferably ± 5%.

The AAV library, preferably, is provided in the form of a kit. Thus, the present invention also relates to a kit comprising a multitude of Adeno-associated virus (AAV) variants, wherein the viral particles of each AAV variant comprise a specific combination of (i) a strain-specific capsid polypeptide and (ii) a peptide inserted into said capsid polypeptide, and wherein said strain-specific capsid polypeptides are derived from at least two different AAV strains.

The terms "Adeno-associated virus" and "AAV" relate to the group of viruses known to the skilled person and containing a short (approx. 4.7 kB) single-stranded DNA and depending in their lytic replication on the presence of an Adenovirus. AAVs are members of the Parvoviridae family of viruses. Also contemplated by the present invention are vectors derived from AAV, i.e. gene transfer vehicles using the capsid polypeptide of AAV to mediate the transfer of recombinant polynucleic acids into target cells. Preferably, the AAV is selected from AAV1 (NC_002077.1, AF063497.1), AAV2 (NC_001401.2), AAV3b (AF028705.1), AAV4 (NC_001829.1, U89790.1), AAV5 (NC_006152.1), AAV6 (AF028704.1), AAV7 (NC_006260.1), AAV8 (NC_006261), AAV9 (AY530579.1), AAVrh.10 (AY243015.1), AAVpo.1 (FJ688147.1), and AAV12 (DQ813647. 1).

The term "AAV variant", as used herein, relates to an AAV corresponding to one of the aforesaid AAVs, however, being non-identical to said AAV. Thus, preferably, the AAV variant comprises a genome comprising at least one mutation compared to said AAV; and/or comprises in its capsid at least one capsid polypeptide comprising at least one amino acid exchange compared to said AAV. Thus, the AAV variant comprises at least one polynucleotide variant as specified herein below as a genome and/or at least one polypeptide variant as specified herein below in its viral capsid. Preferably, the genome of the AAV variant encodes a reporter gene. More preferably, the genomes of all AAV variants of the AAV library encode the same reporter gene. Most preferably, the reporter gene is a fluorescent protein, preferably is green fluorescent protein (GFP). Reporter genes like luciferases, lacZ, and fluorescent proteins (FPs), including yellow FP, blue FP, red FP, and in particular green FP and methods of using them as reporter genes are well known in the art.

The term "capsid polypeptide", as referred to herein, relates to a polypeptide in principle known to the skilled person with the activity of assembly to produce the proteinaceous shell of an AAV particle, also referred to as coat protein or VP protein. It is to be understood that not all AAV capsid polypeptide molecules in a given host cell assemble into AAV capsids. Preferably, at least 25%, at least 50%, at least 75%, at least 85%, at least 90%, at least 95% of all AAV capsid polypeptide molecules assemble into AAV capsids. Suitable assays for measuring this biological activity are described e.g. in Smith-Arica and Bartlett (2001), Curr Cardiol Rep 3(1): 43-49. Preferably, the capsid polypeptide is the capsid polypeptide of AAV1 (Genbank Acc. No: AAD27757.1, GI:4689097), AAV2 (Genbank Acc. No: AAC03780.1, GI:2906023), AAV3b (Genbank Acc. No: AAB95452.1 GI:2766610), AAV4 (Genbank Acc. No: AAC58045.1, GI:2337940), AAV5 (Genbank Acc. No: AAD13756.1, GI:4249658), AAV6 (Genbank Acc NO: AAB95450.1 GI:2766607), AAV7 (Genbank Acc. No: AAN03855.1, GI:22652861), AAV8 (Genbank Acc. No: AAN03857.1, GI:22652864), AAV9 (Genbank Acc. No: AAS99264.1, GI:46487805), AAVrh.10 (Genbank Acc. No: AAO88201.1 GI:29650526), AAVpo.1 (Genbank Acc. No: ACN42940.1 GI:224384439), AAV12 (Genbank Acc. No: ABI16639.1, GI:112379656), or AAV13 (Genbank Acc. No: ABZ10812.1, GI:167047087). Accordingly, preferably, the term "strain-specific capsid polypeptide" relates to a capsid polypeptide having closest similarity, preferably highest % identity values, more preferably a highest % identity value determined as specified herein below, with one of the aforesaid capsid polypeptides. Thus, as used herein, e.g. a capsid polypeptide having closest similarity with the capsid polypeptide of AAV2 is an AAV2-specific capsid polypeptide. In the AAV library of the present invention, the strain-specific capsid polypeptides are derived from at least two different AAV strains. Preferably, the strain-specific capsid polypeptides of the library are derived from at least three, more preferably at least five, even more preferably at least eight, still more preferably at least ten, most preferably at least twelve different AAV strains. Preferably, said strain-specific capsid polypeptides are selected from the list consisting of capsid polypeptides of AAV serotypes 1, 2, 3b, 4, 5, 6, 7, 8, 9, rh.10, po.1 and AAV12.

The term capsid polypeptide, as used herein, preferably includes polypeptide variants of said capsid polypeptides. As used herein, the term "polypeptide variant" relates to any chemical molecule comprising at least one polypeptide or fusion polypeptide as specified elsewhere herein, having the indicated activity, but differing in primary structure from said polypeptide or fusion polypeptide indicated above. Thus, the polypeptide variant, preferably, is a mutein having the indicated activity. Preferably, the polypeptide variant comprises a peptide having an amino acid sequence corresponding to an amino acid sequence of 500 to 1000, more preferably 650 to 800, even more preferably 700 to 750, or, most preferably, 710 to 740 consecutive amino acids comprised in a polypeptide as specified above. Moreover, also encompassed are further polypeptide variants of the aforementioned polypeptides. Such polypeptide variants have at least essentially the same biological activity as the specific polypeptides. Moreover, it is to be understood that a polypeptide variant as referred to in accordance with the present invention shall have an amino acid sequence which differs due to at least one amino acid substitution, deletion and/or addition, wherein the amino acid sequence of the variant is still, preferably, at least 50%, 60%, 70%, 80%, 85%, 90%, 92%, 95%, 97%, 98%, or 99% identical with the amino acid sequence of the specific polypeptide. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the sequence it is compared to for optimal alignment. The percentage is calculated by determining, preferably over the whole length of the polypeptide, the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Polypeptide variants referred to herein may be allelic variants or any other species specific homologs, paralogs, or orthologs. Moreover, the polypeptide variants referred to herein include fragments of the specific polypeptides or the aforementioned types of polypeptide variants as long as these fragments and/or variants have the biological activity as referred to above. Such fragments may be or be derived from, e.g., degradation products or splice variants of the polypeptides. Further included are variants which differ due to posttranslational modifications such as phosphorylation, glycosylation, ubiquitinylation, sumoylation, or myristylation, by including non-natural amino acids, and/or by being peptidomimetics.

Preferably, the capsid polypeptide variant is a chimeric capsid polypeptide. The term "chimeric" is understood by the skilled person to relate to any molecule composed of components originating from at least two different species or strains. Thus, preferably, the capsid polypeptide variant comprises amino acid sequences derived from capsid polypeptides of at least two, preferably at least three AAV strains, more preferably of the AAV strains as specified elsewhere herein. More preferably, the capsid polypeptide variant comprises amino acid sequences of at least five, preferably at least ten, more preferably at least 20 contiguous amino acids derived from capsid polypeptides of at least two, preferably at least three AAV strains, more preferably of the AAV strains as specified elsewhere herein. Preferably, the polynucleotide encoding said chimeric capsid polypeptide was generated by DNA shuffling, a technique which is, in principle, known to the skilled person.

According to the present invention, the capsid polypeptide comprises an inserted peptide. Preferably, the capsid polypeptide comprises said peptide inserted at a site of the capsid polypeptide exposed to the exterior of the AAV capsid, preferably based on structure predictions and/or experimental data. More preferably, the peptide is inserted at a site of the capsid polypeptide exposed to the exterior of the AAV capsid in a manner that does not interfere with the activity of said polypeptide in capsid assembly. Even more preferably, the peptide is inserted into the capsid polypeptide at a position after the amino acid corresponding to amino acid 587 or 588 in the AAV2 capsid polypeptide. Most preferably, the peptide is inserted after amino acid 588 or 590 in case the capsid polypeptide is an AAV1 capsid polypeptide, after amino acid 587 or 588 in case the capsid polypeptide is an AAV2 capsid polypeptide, after amino acid 586 or 588 in case the capsid polypeptide is an AAV3 capsid polypeptide, after amino acid 584 or 586 in case the capsid polypeptide is an AAV4 capsid polypeptide, after amino acid 575 or 577 in case the capsid polypeptide is an AAV5 capsid polypeptide, after amino acid 588 or 590 in case the capsid polypeptide is an AAV6 capsid polypeptide, after amino acid 589 in case the capsid polypeptide is an AAV7 capsid polypeptide, after amino acid 590 in case the capsid polypeptide is an AAV8 capsid polypeptide, after amino acid 588 in case the capsid polypeptide is an AAV9 capsid polypeptide, after amino acid 590 in case the capsid polypeptide is an AAVrh.10 capsid polypeptide, after amino acid 567 or 569 in case the capsid polypeptide is an AAVpo. 1 capsid polypeptide, or is inserted after amino acid 592 or 594 in case the capsid polypeptide is an AAV12 capsid polypeptide.

Preferably, the three amino acids preceding the site into which said peptide is inserted into the capsid polypeptide have been changed to GQS or GQR and/or the three amino acids following the site into which said peptide is inserted have been changed to QAA. Thus, e.g., preferably, in case the capsid polypeptide is an AAV1 capsid polypeptide, amino acids 586 to 588 are GQS or GQR, and, also preferably, the three amino acids directly following the inserted peptide, i.e. the amino acids corresponding to amino acids 589 to 591 of the unmodified AAV capsid polypeptide are QAA; or amino acids 588 to 590 are GQS or GQR, and, also preferably, the three amino acids directly following the inserted peptide, i.e. the amino acids corresponding to amino acids 592 to 594 of the unmodified AAV capsid polypeptide are QAA. In preferred embodiments, the insertions sites correspond to the amino acids and optionally have the modified flanking amino acids as shown in Table 1:

**Table 1: Preferred insertion site/flanking amino acids combinations. Amino acids between which insertion of a peptide is preferred are separated by a "-"; Position indications are relative to the amino acid sequence of the respective wildtype capsid polypeptide as indicated elsewhere herein.**

| | **Variant 1 insertion sites (OIS):** | | **Variant 2 insertion sites (NIS):** | |
|---|---|---|---|---|
| **Serotype** | **Insertion Site** | **Modified Amino Acids (SEQ ID NO:)** | **Insertion Site** | **Modified Amino Acids (SEQ ID NO:)** |
| **AAV 1** | D590_P591 | STD-PAT (15) | **S588_T589** | **SSS-TDP (26)** |
| **AAV2** | R588_Q589 | GNR-QAA (16) | **N587_R588** | **RGN-RQA (27)** |
| **AAV3b** | S586_S587 | LQS-SNT (17) | **N588_T589** | **SSN-TAP (28)** |
| **AAV4** | S584_N585 | DQS-NSN (18) | **S586_N587** | **SNS-NLP (29)** |
| **AAV5** | S575_S576 | NQS-STT (19) | **T577_T578** | **SST-TAP (30)** |
| **AAV6** | D590_P591 | STD-PAT (15) | **S588_T589** | **SSS-TDP (26)** |
| **AAV7** | N589_T590 | AAN-TAA (20) | - | - |
| **AAV8** | N590_T591 | QQN-TAP (21) | - | - |
| **AAV9** | Q588_A589 | SAQ-AQA (22) | - | - |
| **AAVrh10** | N590_A591 | QQN-AAP (23) | - | - |
| **AAVpo.1** | N567_S568 | NQN-SNT (24) | **N569_T570** | **NSN-THP (31)** |
| **AAV12** | N592_A593 | NQN-ATT (25) | **T594_T595** | **NAT-TAP (32)** |

The term "library" is known to the skilled person as relating to a collection of compounds having a set of features in common, however differing in at least one determinable property. E.g., in a virus library, the member viruses are derived from the viruses of the same species, but differ e.g. in a surface-exposed peptide. In the AAV library of the present invention, specific combinations of (i) a strain-specific AAV capsid polypeptide and (ii) a peptide inserted into said capsid polypeptide are comprised and the strain-specific capsid polypeptides are derived from at least two different AAV strains. The viral particles of each AAV variant in the library comprise a specific combination of (i) a strain-specific capsid polypeptide and (ii) a peptide inserted into said capsid polypeptide. Preferably, each AAV variant in the library comprises exactly one specific combination of (i) a strain-specific capsid polypeptide and (ii) a peptide inserted into said capsid polypeptide. Thus, preferably, each AAV particle comprised in said AAV library comprises exactly one specific combination of (i) a strain-specific capsid polypeptide and (ii) a peptide inserted into said capsid polypeptide. Accordingly, preferably, the AAV library comprises said AAV variants in an isolated manner, i.e. preferably, the AAV library does not comprise a mixture of AAV variants. The term "in an isolated manner" relates to a manner of providing said AAV variants such that the AAV variants are spatially separated, e.g. in different test tubes, in different cell culture plates, or in different wells of a cell culture plate. More preferably, said AAV library comprises said AAV variants in an ordered array, preferably in one or more multi-well plate(s). The term "ordered array" is understood by the skilled person; preferably, the term relates to an array which allows identification of a specific capsid polypeptide/peptide combination used in a specific infection solely based on the information on the position of the respective AAV particles used in a spatial arrangement. Preferably, the library additionally comprises naturally occurring AAVs as specified herein above, e.g. as controls. Also preferably, the library comprises naturally occurring and non-naturally occurring AAV variants. More preferably, the library comprises non-naturally occurring AAV variants. Most preferably, the library consists of non-naturally occurring AAV variants. Thus, preferably, the AAV variants, the AAV polypeptides, the polynucleotides, and/or the cloning cells of the present invention are non-naturally occurring. Preferably, the AAV library comprises each type of variant AAV in at least two different titers.

The term "peptide", as used herein, relates to a molecule comprising at least two amino acids connected by a peptide bond. Preferably, the peptide has a length of from 3 to 20 amino acids, more preferably of from 4 to 12 amino acids, more preferably of from 7 to 9 amino acids. Preferably, all amino acids comprised in the peptide are alpha-amino acids, more preferably are L-alpha-amino acids, most preferably are proteinogenic amino acids, i.e. are amino acids included in a polypeptide by protein biosynthesis. Preferably, all bonds between the amino acids comprised in the peptide and the bonds between the peptide and the capsid polypeptide are peptide bonds. Preferably, the peptide is included in the structure of the capsid polypeptide during protein biosynthesis, more preferably by expression from a gene encoding the capsid polypeptide including the peptide. Thus, preferably, the capsid polypeptide and the peptide are encoded by a contiguous open reading frame. Preferably, the peptide additionally comprises a small amino acid, preferably independently selected from G and A, at the N-terminus and/or at the C-terminus, i.e. preferably, intervening between the sequence of the capsid polypeptide and the peptide. More preferably, the peptide additionally comprises a G at the N-terminus and an A at the C-terminus. The amino acid sequence of the peptide can be any amino acid sequence, e.g. a random sequence from a peptide library. Preferably, the library comprises each peptide comprised therein in combination with each strain-specific capsid polypeptide comprised therein. Preferably, the AAV library comprises at least 50, preferably at least 100, more preferably at least 200 non-identical peptides inserted into AAV capsid polypeptides. Preferably, at least one inserted peptide is comprised in the AAV library in combination with each strain-specific capsid polypeptide comprised in the AAV library. More preferably, at least five, preferably at least ten, more preferably at least 50, even more preferably at least 100 inserted peptides are comprised in the AAV library in combination with each strain-specific capsid polypeptide comprised in the AAV library. Even more preferably, all combinations of at least 100 inserted peptides with at least 12 strain-specific capsid polypeptides are comprised in the AAV library. Most preferably, the AAV library comprises each inserted peptide in combination with each strain-specific capsid polypeptide.

Preferably, the amino acid sequence of the peptide is a sequence known or suspected to assist or to mediate functional transduction of an AAV variant comprising said amino acid sequence to a target cell of interest, wherein the term "functional transduction" includes all aspects of AAV particle entry having an impact on reporter gene expression, i.e., e.g., preferably, binding, uptake, intracellular processing, nuclear import, uncoating and transgene expression. Preferably, the amino acid sequence of the peptide is a sequence known or suspected to assist or to mediate binding of an AAV variant comprising said amino acid sequence to a target cell of interest. An amino acid sequence known to assist or mediate binding to a target cell of interest may be a sequence identified in a previous screening assay or a variant thereof. An amino acid sequence suspected to assist or mediate binding to a target cell of interest may be a sequence or a variant thereof identified by structure prediction or by alignment of ligands of said target cell. Preferably, the peptide comprises a motif NXXR (SED ID NO:1), preferably NXXRXXX (SEQ ID NO:2) wherein in both SEQ ID NO:1 and SEQ ID NO:2, X is independently selected any amino acid, preferably independently selected from the list consisting of all proteinogenic amino acids. Thus, preferably, at least one specific combination per AAV strain comprises an amino acid motif NXXR (SED ID NO:1), preferably comprises an amino acid motif NXXRXXX (SED ID NO:2). More preferably, at least 10%, preferably at least 20% of the specific combinations comprised in the AAV library comprise a motif NXXR, preferably NXXRXXX (SED ID NO:2). Even more preferably, at least one of the specific combinations per AAV strain comprises a motif selected from the list consisting of CDCRGDCFC (SEQ ID NO:3), NDVRSAN (SEQ ID NO:4), NDVRAVS (SEQ ID NO:5), MPLGAAG (SEQ ID NO:6), NYSRGVD (SEQ ID NO:7), and NEARVRE (SEQ ID NO:8). As detailed elsewhere herein, the peptides are preferably flanked by small amino acids, more preferably a G at the N-terminus and an A at the C-terminus. Thus, preferably, the peptides comprised in the AAV capsid polypeptide are selected from amino acid sequences GCDCRGDCFCA (SEQ ID NO:9), GNDVRSANA (SEQ ID NO:10), GNDVRAVSA (SEQ ID NO:11), GMPLGAAGA (SEQ ID NO:12), GNYSRGVDA (SEQ ID NO:13), and GNEARVREA (SEQ ID NO:14). Preferably, the library comprises at least one specific combination per strain-specific capsid polypeptide with a peptide comprising, preferably consisting of, an amino acid sequence selected from the list consisting of SEQ ID NOs: 3 to 14. More preferably, the library comprises specific combinations per strain-specific capsid polypeptide with at least two, preferably at least three, more preferably at least four, more preferably at least five, even more preferably all six peptides comprising, preferably consisting of, amino acid sequences selected from the list consisting of SEQ ID NOs: 3 to 8; and/or comprises specific combinations per strain-specific capsid polypeptide with at least two, preferably at least three, more preferably at least four, more preferably at least five, even more preferably all six peptides comprising, preferably consisting of, amino acid sequences selected from the list consisting of SEQ ID NOs: 9 to 14. Thus, for example, in case the library comprises strain-specific capsid polypeptides of AAV strains 1, 2, and 4, it, preferably, comprises the following combinations of capsid polypeptide/peptide: AAV1/SEQ ID NO:9, AAV1/SEQ ID NO:10, AAV1/SEQ ID NO:11, AAV1/SEQ ID NO:12, AAV1/SEQ ID NO:13, AAV1/SEQ ID NO:14, AAV2/SEQ ID NO:9, AAV2/SEQ ID NO:10, AAV2/SEQ ID NO:11, AAV2/SEQ ID NO:12, AAV2/SEQ ID NO:13, AAV2/SEQ ID NO:14, AAV4/SEQ ID NO:9, AAV4/SEQ ID NO:10, AAV4/SEQ ID NO:11, AAV4/SEQ ID NO:12, AAV4/SEQ ID NO:13, and AAV4/SEQ ID NO:14.

Advantageously, it was found in the work underlying the present invention that including in an AAV library further AAV strains besides the most widely used strain AAV2 improves the probability of finding a variant capsid polypeptide suitable for infecting a specific target cell of interest. Moreover, by maintaining each specific capsid polypeptide/peptide variant in an isolated manner, repeated rounds of infection/replication/isolation can be avoided. Accordingly, according to the present invention, a suitable AAV strain can usually be identified within one round of infection, i.e. within less than three days. Furthermore, by using the specific insertion sites and the specific modifications of the sequences surrounding the inserted peptides within the insertion site, the peptides can be optimally presented. Furthermore, the peptides disclosed herein were found to mediate infection of a large variety of target cells at a high frequency.

The definitions made above apply mutatis mutandis to the following. Additional definitions and explanations made further below also apply for all embodiments described in this specification mutatis mutandis.

The present invention also relates to a method for identifying an AAV variant infecting a target cell of interest comprising
a) providing a library of AAV variants comprising each AAV variant in an isolated manner, wherein each AAV variant of said library of AAV variants comprises a specific combination of (i) a strain-specific capsid polypeptide and (ii) a peptide inserted into said capsid polypeptide and wherein said strain-specific capsid polypeptides are derived from at least two different AAV strains;
b) contacting each of said AAV variants individually with said target cell of interest;
c) determining AAV infection of said target cells of interest; and
d) based on the result of step c), identifying a variant AAV infecting a target cell of interest.

The method of the present invention, preferably, is an in vitro method. Moreover, it may comprise steps in addition to those explicitly mentioned above. For example, further steps may relate, e.g., to providing candidate peptides assisting in or mediating AAV infection of the target cell of interest for step a), or contacting said AAV variants with said target cell of interest at a second titer in step b). Moreover, one or more, more preferably all of said steps may be performed by automated equipment. Preferably, the library of AAV variants used in the method of the present invention is the library of AAV variants as specified herein above.

The term "cell", as used herein, is understood by the skilled person as relating to the smallest structural unit in biology with the principal ability of self-replication. Thus, the term includes archeal, prokaryotic, and eukaryotic cells. The term "cloning cell" relates to a cell capable of stably maintaining an AAV genome, and/or a reporter gene, preferably by replication as a plasmid, e.g. in a bacterial cell, in particular an *Escherichia coli* cell; or by maintenance and/or replication of the AAV genome in said cell, in particular a human cell. Preferably, the cloning cell is suitable for producing AAV particles, i.e. is a host cell as specified below. The term "target cell" relates to a cell known or suspected to be infectible by an AAV. Preferably, the target cell is a cell of a subject or a cultured cell derived therefrom. Preferably, the target cell is not a skeletal muscle cell, is not a neuron, is not a vascular smooth muscle cell, is not a hepatocyte, is not a synoviocyte, is not an airway epithelial cell, and/or is not an astrocyte. The term "host cell" is known to the skilled person and relates to a cell allowing packaging of an AAV genome into a viral particle to occur. Thus, preferably, the host cell is suitable for producing AAV particles. As used herein, a host cell may be a packaging cell, i.e. a cell comprising all components required for packaging an AAV genome into an AAV capsid. As will be understood by the skilled person, a cloning cell can be a host cell and can be a packaging cell, but does not have to be, e.g. in case the cell is an *E. coli* cell. Also, a host cell can be a target cell, but does not have to be, e.g. in case the host cell lacks a receptor for AAV infection. Likewise, a target cell may be a host cell, but does not have to be, e.g. in case the cell is infectible, but is not permissive for AAV replication or packaging. Preferably, the host cell and/or the target cell are/is a mammalian cell, more preferably, a human cell. Preferably, the host cell and/or the target cell are/is a cell maintained in vitro in a suitable cultivation medium.

The term "determining AAV infection" of a target cell, as used herein, refers to determining at least one characteristic feature of an AAV in said target cell. A characteristic feature is a feature which characterizes the presence of an AAV in a target cell, preferably is an AAV constituent or a derivative thereof, preferably is an AAV genome. Characteristic features preferably include, e.g., presence of viral capsid polypeptides, presence of viral genomes, production of viral progeny, expression of a reporter gene, and the like. Said features may be determined qualitatively, semi-quantitatively, or quantitatively. Preferably, determination is quantitative. Preferably, determining AAV infection comprises determining, preferably determining semi-quantitatively, more preferably determining quantitatively, expression of a reporter gene comprised on the genome of said variant AAV. Preferably, the genomes of all AAV variants of the AAV library encode the same reporter gene. Preferably, the reporter gene is a reporter gene as specified herein above; thus, more preferably, the reporter gene is a gene encoding a fluorescent protein, most preferably is a reporter gene encoding GFP. Preferably, determining AAV infection comprises determining for each AAV variant the number of target cells of interest infected by said AAV variant. Also preferably, determining AAV infection comprises determining for each AAV variant the expression level of said reporter gene in target cells of interest infected by said AAV variant.

According to the invention, a variant AAV infecting a target cell of interest is identified based on the result of the steps as specified herein. As will be understood by the skilled person, the criteria which variant AAV is considered to infect a target cell of interest will depend on a variety of factors, in particular on the target cell of interest. Preferably, identifying a variant AAV infecting a target cell of interest comprises the steps as specified above and the further step of determining infection of said target cell in a control infection; and comparing infection by said control infection to infection of said target cell by said variant AAV. Appropriate control infections are known to the skilled person and include, preferably, a mock-infection, i.e. a pseudo infection in the absence of AAV particles; and/or a control infection with a parental AAV, i.e. an AAV comprising a capsid polypeptide lacking an inserted peptide. Preferably, in such case, a variant AAV infecting a target cell of interest is identified in case infection by the variant AAV is higher than in the control infection, more preferably in case infection by the variant AAV is statistically significant higher than in the control infection.

Preferably, the method of the present invention further comprises a step of contacting each of the AAV variants individually with a target cell of non-interest; a step of determining AAV infection of said target cells of non-interest; and a step of based on the results of the aforesaid steps, identifying a variant AAV infecting a target cell of interest but not infecting a target cell of non-interest. Thus, preferably, the method of the present invention is a method for identifying a variant AAV differentially infecting a target cell of interest, but not a target cell of non-interest, or is a method for identifying a variant AAV specifically infecting a target cell of interest.

Preferably, the target cell of interest is a cell of a subject involved in disease, causing disease, or suitable for treating disease. As used herein, the term "subject" relates to a metazoan organism comprising at least one target cell. Preferably, the subject is an animal, more preferably a mammal, most preferably a human being. Preferably, the subject is a subject suffering from disease, a subject at risk of suffering from disease, or a subject suspected to be suffering from disease. As will be understood, a subject at risk of suffering from a disease, preferably, is a subject having been assigned at least one risk factor for suffering from said disease; and a subject suspected to be suffering from disease may be a subject suspected to suffer from disease based on the frequency of a disease in the population and, accordingly, may be an apparently healthy subject. Preferably, said disease is a metabolic disease such as hemophilia type A or B, a disease that is characterized by a functional deficiency of affected cells due to mutation(s) of the genome such as cystic fibrosis, a disease involving cell and/or tissue damage such as neurodegenerative disorders, myopathies or cardiomyopathies, or an infectious disease such as infection with human immunodeficiency virus (HIV), any of hepatitis viruses types A to E, or parasites such as Plasmodium. Preferably, said disease is not an arthritic disease.

The present invention further relates to a library of polynucleotides encoding the AAV variants of the AAV library according to the present invention.

The term "polynucleotide", as used herein, refers to a linear or circular nucleic acid molecule. The polynucleotide of the present invention shall be provided, preferably, either as an isolated polynucleotide (i.e. isolated from its natural context) or in genetically modified form, preferably comprising at least one heterologous sequence. The term encompasses single- as well as doublestranded polynucleotides. Moreover, comprised are also chemically modified polynucleotides including naturally occurring modified polynucleotides such as glycosylated or methylated polynucleotides or artificially modified derivatives such as biotinylated polynucleotides.

The polynucleotide of the present invention encodes a variant AAV capsid polypeptide of the present invention. Preferably, the polynucleotide comprises a sequence encoding an AAV capsid polypeptide as specified herein, wherein a nucleic acid sequence encoding a specific combination of (i) a strain-specific capsid polypeptide and (ii) a peptide inserted into said capsid polypeptide, preferably replacing the nucleic acid sequence encoding the naturally occurring capsid polypeptide. This can be accomplished in various ways using cloning methods known to the skilled person.

The polynucleotides of the invention have the activity of encoding a modified AAV capsid polypeptide. Methods for testing whether a given polynucleotide has the aforesaid activity are known in the art. E.g., preferably, a candidate polynucleotide of interest is introduced into a packaging cell and production of AAV particles is determined. Whether a polynucleotide encodes a variant capsid polypeptide can be established e.g. by sequencing the gene encoding said capsid polypeptide and comparing the sequence obtained to the corresponding wildtype sequence.

Moreover, the term "polynucleotide" as used in accordance with the present invention preferably further encompasses variants of the aforementioned specific polynucleotides. Said variants may represent strain or clonal variants of the polynucleotide of the present invention. The polynucleotide variants, preferably, comprise a nucleic acid sequence characterized in that the sequence can be derived from the aforementioned specific nucleic acid sequences by at least one nucleotide substitution, addition and/or deletion whereby the variant nucleic acid sequence shall still have the activity of encoding a variant AAV capsid polypeptide as specified above. A polynucleotide comprising a fragment of any of the aforementioned nucleic acid sequences is also encompassed as a polynucleotide of the present invention. The fragment shall have the activity of encoding an AAV with a modified capsid polypeptide as specified above. Preferably, said polynucleotide variant is a naturally occurring variant of the specific polynucleotide. More preferably, said polynucleotide variant is a non-naturally occurring variant of the specific polynucleotide. Polynucleotide variants also encompass polynucleotides comprising a nucleic acid sequence which is capable of hybridizing to the aforementioned specific polynucleotides, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6x sodium chloride/sodium citrate (= SSC) at approximately 45°C, followed by one or more wash steps in 0.2x SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1x to 5x SSC (pH 7.2). If organic solvent is present in the abovementioned buffer, for example 50% formamide, the temperature under standard conditions is approximately 42°C. The hybridization conditions for DNA:DNA hybrids are preferably for example 0.1x SSC and 20°C to 45°C, preferably between 30°C and 45°C. The hybridization conditions for DNA:RNA hybrids are preferably, for example, 0.1x SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, polynucleotide variants are obtainable by PCR-based techniques such as mixed oligonucleotide primer-based amplification of DNA, i.e. using degenerated primers derived from conserved domains of a polypeptide of the present invention. Conserved domains of a polypeptide may be identified by a sequence comparison of the nucleic acid sequence of the polynucleotide or the amino acid sequence of the polypeptide of the present invention with sequences of other organisms. As a template, DNA or cDNA from a parvovirus, in particular an AAV, may be used. Further, variants include polynucleotides comprising nucleic acid sequences which are at least 70%, at least 75%, at least 80%, at least 85%, at least 90%, at least 95%, at least 98% or at least 99% identical to the specifically indicated nucleic acid sequences. Moreover, also encompassed are polynucleotides which comprise nucleic acid sequences encoding AAV polypeptides having amino acid sequences which are at least 70%, at least 75%, at least 80%, at least 85%, at least 90%, at least 95%, at least 98% or at least 99% identical to the amino acid sequences as specified above. Percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit (Needleman and Wunsch (J. Mol. Biol. 48; 443-453 (1970)) and Smith and Waterman (Adv. Appl. Math. 2; 482-489 (1981)), which are part of the GCG software packet (Genetics Computer Group, 575 Science Drive, Madison, Wisconsin, USA 53711 (1991)), are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the specifically indicated nucleic acid sequences is also encompassed as a variant polynucleotide of the present invention, wherein the fragment shall still encode a variant AAV capsid polypeptide which still has the activity as specified. A fragment as meant herein, preferably, comprises at least 1000, more preferably at least 2000, most preferably at least 2100 consecutive nucleotides of any one of the specific nucleic acid sequences or encodes an amino acid sequence comprising at least 500, more preferably at least 600, most preferably at least 700 consecutive amino acids of any one of the specific amino acid sequences.

The polynucleotides of the present invention either consist of the aforementioned nucleic acid sequences or comprise the aforementioned nucleic acid sequences. Thus, they may contain further nucleic acid sequences as well. Specifically, the polynucleotides of the present invention may encode further polypeptides, e.g. reporter polypeptides, and/or fusion polypeptides wherein one partner of the fusion protein is a polypeptide being encoded by a nucleic acid sequence recited above. Such fusion proteins may comprise as additional part polypeptides for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and comprise FLAG-tags, 6-histidine-tags, MYC-tags and the like.

Preferably, the polynucleotide or at least one part thereof encodes a chimeric capsid polypeptide as specified elsewhere herein. Thus, preferably, the polynucleotide or at least one part thereof is generated by DNA shuffling.

The present invention further relates to a library of cloning cells comprising the AAV variants of the AAV library according to the present invention and/or comprising polynucleotides encoding the same.

The term "cloning cell", as specified herein above, relates to a cell capable of stably maintaining an AAV genome, preferably by replication as a plasmid, e.g. in a bacterial cell, in particular an *Escherichia coli* cell; or by maintenance and/or replication of the AAV genome in said cell, in particular a human cell. As will be understood by the skilled person, additional nucleic acid sequences may be required for stable maintenance of an AAV genome in a cell, e.g. a bacterial origin of replication and/or a selectable marker. Thus, preferably, the polynucleotide encoding an AAV variant of the present invention, preferably, is comprised in a vector.

The term "vector", preferably, encompasses phage, plasmid, viral or retroviral vectors as well artificial chromosomes, such as bacterial or yeast artificial chromosomes. Moreover, the term also relates to targeting constructs which allow for random or site-directed integration of the targeting construct into genomic DNA. Such target constructs, preferably, comprise DNA of sufficient length for either homologous or heterologous recombination as described in detail below. The vector encompassing the polynucleotide of the present invention, preferably, further comprises selectable markers for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerenes. Alternatively, a plasmid vector may be introduced by heat shock or electroporation techniques. Should the vector be a virus, it may be packaged in vitro using an appropriate packaging cell line or alternative packaging systems prior to application to host cells.

More preferably, in the vector of the invention, the polynucleotide is operatively linked to expression control sequences allowing expression in eukaryotic cells or isolated fractions thereof. Expression of said polynucleotide comprises transcription of the polynucleotide, preferably into a translatable mRNA. Regulatory elements ensuring expression in eukaryotic cells, preferably mammalian cells, are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Possible regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells. Preferably, the regulatory element comprises the AAV p40 promoter. Moreover, inducible expression control sequences may be used in an expression vector encompassed by the present invention. Suitable expression control sequences are well known in the art. Beside elements which are responsible for the initiation of transcription such regulatory elements may also comprise transcription termination signals, such as the SV40-poly-A site, the tk-poly-A site, or the AAV poly-A site downstream of the polynucleotide. Methods which are well known to those skilled in the art can be used to construct the polynucleotides of the invention; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

Furthermore, the present invention relates to the use of an AAV library according to the present invention, a method according to the present invention, a library of polynucleotides according to the present invention, and/or a library of cloning cells according to the present invention for identifying an AAV variant infecting a target cell of interest.

In view of the above, the following embodiments are particularly preferred:
1. An Adeno-associated virus (AAV) library comprising a multitude of Adeno-associated virus (AAV) variants, wherein the viral particles of each AAV variant comprise a specific combination of (i) a strain-specific capsid polypeptide and (ii) a peptide inserted into said capsid polypeptide, and wherein said strain-specific capsid polypeptides are derived from at least two different AAV strains.
2. The AAV library of embodiment 1, wherein said strain-specific capsid polypeptides are derived from at least three, preferably at least five, more preferably at least eight, still more preferably at least ten, most preferably at least twelve different AAV strains.
3. The AAV library of embodiment 1 or 2, wherein said AAV strains are selected from the list consisting of AAV serotypes 1, 2, 3b, 4, 5, 6, 7, 8, 9, rh.10, po.1 and AAV12.
4. The AAV library of any one of embodiments 1 to 3, wherein said capsid polypeptides derived from at least two different AAV strains are selected from the list consisting of AAV1 (Genbank Acc No NC_002077.1, AF063497.1), AAV2 (Genbank Acc No NC_001401.2), AAV3b (Genbank Acc No AF028705.1), AAV4 (Genbank Acc No NC_001829.1, U89790.1), AAV5 (Genbank Acc No NC_006152.1), AAV6 (Genbank Acc No AF028704.1), AAV7 (Genbank Acc No NC_006260.1), AAV8 (Genbank Acc No NC_006261), AAV9 (Genbank Acc No AY530579.1), AAVrh.10 (Genbank Acc No AY243015.1), AAVpo.1 (Genbank Acc No FJ688147.1), AAV12 (Genbank Acc No DQ813647.1).
5. The AAV library of any one of embodiments 1 to 4, wherein said peptides have a length of from 3 to 20 amino acids, preferably of from 4 to 12 amino acids, more preferably of from 7 to 9 amino acids.
6. The AAV library of any one of embodiments 1 to 5, wherein at least one of said specific combinations comprises a motif NXXR (SEQ ID NO:1), preferably NXXRXXX (SEQ ID NO:2).
7. The AAV library of any one of embodiments 1 to 6, wherein at least one of said specific combinations per AAV strain comprises a motif NXXR (SEQ ID NO:1), preferably NXXRXXX (SEQ ID NO: 2).
8. The AAV library of any one of embodiments 1 to 7, wherein at least 10%, preferably at least 20% of said specific combinations comprised in said AAV library comprise a motif NXXR (SEQ ID NO:1), preferably NXXRXXX (SEQ ID NO:2).
9. The AAV library of any one of embodiments 1 to 8, wherein at least one of said specific combinations per AAV strain comprises a motif selected from the list consisting of CDCRGDCFC (SEQ ID NO:3), NDVRSAN (SEQ ID NO:4), NDVRAVS (SEQ ID NO:5), MPLGAAG (SEQ ID NO:6), NYSRGVD (SEQ ID NO:7), and NEARVRE (SEQ ID NO:8).
10. The AAV library of any one of embodiments 1 to 9, wherein said peptides additionally comprise a small amino acid, preferably independently selected from G and A, at the N-terminus and/or at the C-terminus.
11. The AAV library of any one of embodiments 1 to 10, wherein said peptides additionally comprise a G at the N-terminus and an A at the C-terminus.
12. The AAV library of any one of embodiments 1 to 11, wherein said peptide is inserted at a site of the capsid polypeptide exposed to the exterior of the AAV capsid.
13. The AAV library of any one of embodiments 1 to 12, wherein said peptide is inserted at a site of the capsid polypeptide exposed to the exterior of the AAV capsid in a manner that does not interfere with the activity of said polypeptide in capsid assembly.
14. The AAV library of any one of embodiments 1 to 13, wherein said peptide is inserted into the capsid polypeptide at a position after the amino acid corresponding to amino acid 587 or 588 in the AAV2 capsid polypeptide.
15. The AAV library of any one of embodiments 1 to 14, wherein said peptide is inserted
   after amino acid 588 or 590 in case the capsid polypeptide is an AAV1 capsid polypeptide,
   after amino acid 587 or 588 in case the capsid polypeptide is an AAV2 capsid polypeptide,
   after amino acid 586 or 588 in case the capsid polypeptide is an AAV3 capsid polypeptide,
   after amino acid 584 or 586 in case the capsid polypeptide is an AAV4 capsid polypeptide,
   after amino acid 575 or 577 in case the capsid polypeptide is an AAV5 capsid polypeptide,
   after amino acid 588 or 590 in case the capsid polypeptide is an AAV6 capsid polypeptide,
   after amino acid 589 in case the capsid polypeptide is an AAV7 capsid polypeptide,
   after amino acid 590 in case the capsid polypeptide is an AAV8 capsid polypeptide,
   after amino acid 588 in case the capsid polypeptide is an AAV9 capsid polypeptide,
   after amino acid 590 in case the capsid polypeptide is an AAVrh. 10 capsid polypeptide,
   after amino acid 567 or 569 in case the capsid polypeptide is an AAVpo. 1 capsid polypeptide, or
   after amino acid 592 or 594 in case the capsid polypeptide is an AAV12 capsid polypeptide.
16. The AAV library of any one of embodiments 1 to 15, wherein the three amino acids preceding the site into which said peptide is inserted have been changed to GQS or GQR and/or wherein the three amino acids following the site into which said peptide is inserted have been changed to QAA.
17. The AAV library of any one of embodiments 1 to 16, wherein said capsid polypeptides comprising an inserted peptide are encoded on the genome of said AAV particles.
18. The AAV library of any one of embodiments 1 to 17, wherein said AAV library comprises said AAV variants in an isolated manner.
19. The AAV library of any one of embodiments 1 to 18, wherein said AAV library comprises said AAV variants in an ordered array, preferably in one or more multi-well plate(s).
20. The AAV library of any one of embodiments 1 to 19, wherein said AAV library comprises at least 50, preferably at least 100, more preferably at least 200 non-identical peptides inserted into AAV capsid polypeptides.
21. The AAV library of any one of embodiments 1 to 20, wherein at least one inserted peptide is comprised in the AAV library in combination with each strain-specific capsid polypeptide comprised in the AAV library.
22. The AAV library of any one of embodiments 1 to 21, wherein at least five, preferably at lest ten, more preferably at least 50, even more preferably at least 100 inserted peptides are comprised in the AAV library in combination with each strain-specific capsid polypeptide comprised in the AAV library.
23. The AAV library of any one of embodiments 1 to 22, wherein all combinations of at least 100 inserted peptides with at least 12 strain-specific capsid polypeptides are comprised in the AAV library.
24. The AAV library of any one of embodiments 1 to 23, wherein said AAV library comprises each inserted peptide in combination with each strain-specific capsid polypeptide.
25. The AAV library of any one of embodiments 1 to 24, wherein said AAV variants are non-naturally occurring AAV variants.
26. The AAV library of any one of embodiments 1 to 25, wherein the genomes of said AAV variants encode a reporter gene.
27. The AAV library of any one of embodiments 1 to 26, wherein the genomes of all AAV variants of the AAV library encode the same reporter gene.
28. The AAV library of embodiment 26 or 27, wherein said reporter gene is a fluorescent protein, preferably is green fluorescent protein (GFP).
29. The AAV library of any one of embodiments 1 to 28, wherein said AAV library comprises each type of variant AAV in at least two different titers.
30. A method for identifying an AAV variant infecting a target cell of interest comprising
   a) providing a library of AAV variants comprising each AAV variant in an isolated manner, wherein each AAV variant of said library of AAV variants comprises a specific combination of (i) a strain-specific capsid polypeptide and (ii) a peptide inserted into said capsid polypeptide and wherein said strain-specific capsid polypeptides are derived from at least two different AAV strains;
   b) contacting each of said AAV variants individually with said target cell of interest;
   c) determining AAV infection of said target cells of interest; and
   d) based on the result of step c), identifying a variant AAV infecting a target cell of interest.
31. The method of embodiment 30, wherein the genomes of said AAV variants encode a reporter gene.
32. The method of embodiment 30 or 31, wherein the genomes of all AAV variants of the AAV library encode the same reporter gene.
33. The method of any one of embodiments 30 to 32, wherein said reporter gene is green fluorescent protein (GFP).
34. The method of any one of embodiments 30 to 33, wherein step c) comprises determining expression of said reporter gene in said target cells.
35. The method of any one of embodiments 30 to 34, wherein step c) comprises determining for each AAV variant the number of target cells of interest infected by said AAV variant.
36. The method of any one of embodiments 30 to 35, wherein step c) comprises determining for each AAV variant the expression level of said reporter gene in target cells of interest infected by said AAV variant.
37. The method of any one of embodiments 30 to 36, wherein said library of AAV variants is the AAV library according to any one of embodiments 1 to 23.
38. The method of any one of embodiments 30 to 37, wherein said strain-specific capsid polypeptides are derived from at least three, preferably at least five, more preferably at least eight, still more preferably at least ten, most preferably at least twelve different AAV strains.
39. The method of any one of embodiments 30 to 38, wherein said AAV strains are selected from the list consisting of AAV serotypes 1, 2, 3b, 4, 5, 6, 7, 8, 9, rh.10, po.1 and AAV12.
40. The method of any one of embodiments 30 to 39, wherein said capsid polypeptides derived from at least two different AAV strains are selected from the list consisting of AAV1 (NC_002077.1, AF063497.1), AAV2 (NC_001401.2), AAV3b (AF028705.1), AAV4 (NC_001829.1, U89790.1), AAV5 (NC_006152.1), AAV6 (AF028704.1), AAV7 (NC_006260.1), AAV8 (NC_006261), AAV9 (AY530579.1), AAVrh.10 (AY243015.1), AAVpo.1 (FJ688147. 1), AAV12 (DQ813647. 1).
41. The method of any one of embodiments 30 to 40, wherein said peptides have a length of from 3 to 20 amino acids, preferably of from 4 to 12 amino acids, more preferably of from 7 to 9 amino acids.
42. The method of any one of embodiments30 to 41, wherein said peptide is inserted at a site of the capsid polypeptide exposed to the exterior of the AAV capsid.
43. The method of any one of embodiments 30 to 42, wherein said capsid polypeptides comprising an inserted peptide are encoded on the genome of said AAV particles.
44. The method of any one of embodiments 30 to 43, wherein said AAV library comprises said AAV variants in an ordered array, preferably in one or more multi-well plate(s).
45. The method of any one of embodiments 30 to 44, wherein said AAV library comprises at least 50, preferably at least 100, more preferably at least 200 non-identical peptides inserted into AAV capsid polypeptides.
46. The method of any one of embodiments 30 to 45, wherein at least one inserted peptide is comprised in the AAV library in combination with each strain-specific capsid polypeptide comprised in the AAV library.
47. The method of any one of embodiments 30 to 46, wherein at least five, preferably at lest ten, more preferably at least 50, even more preferably at least 100 inserted peptides are comprised in the AAV library in combination with each strain-specific capsid polypeptide comprised in the AAV library.
48. The method of any one of embodiments 30 to 47, wherein all combinations of at least 100 inserted peptides with at least 12 strain-specific capsid polypeptides are comprised in the AAV library.
49. The method of any one of embodiments 30 to 48, wherein said AAV library comprises each inserted peptide in combination with each strain-specific capsid polypeptide.
50. The method of any one of embodiments 30 to 49, wherein method comprises contacting each type of variant AAV with said target cell of interest in at least two different titers.
51. The method of any one of embodiments 30 to 50, wherein said method further comprises steps b1) contacting each of said AAV variants individually with a target cell of non-interest; and c1) determining AAV infection of said target cells of non-interest; and comprises step d) based on the results of steps c) and c1), identifying a variant AAV infecting a target cell of interest but not infecting a target cell of non-interest.
52. A library of polynucleotides encoding the variant AAV capsid polypeptides of the AAV library according to any one of embodiments 1 to 29.
53. The library of polynucleotides of embodiment 52, wherein each polynucleotide encoding a specific variant AAV capsid polypeptide is comprised in said library of polynucleotides in an isolated manner.
54. A library of cloning cells comprising the AAV variants of the AAV library according to any one of embodiments 1 to 29 and/or comprising polynucleotides encoding the same.
55. The library of cloning cells of embodiment 54, wherein each AAV variant is comprised in said library of cloning cells in an isolated manner.
56. The library of cloning cells of embodiment 54 or 55, wherein said cloning cells are bacterial cells or host cells, preferably are host cells.
57. Use of an AAV library according to any one of embodiments 1 to 29, a method according to any one of embodiments 30 to 51, a library of polynucleotides according to embodiment 52 or 53, and/or a library of cloning cells according to embodiment 54 or 56 for identifying an AAV variant infecting a target cell of interest.
58. An AAV capsid polypeptide comprising an inserted peptide comprising an amino acid sequence motif NXXR (SEQ ID NO: 1), preferably NXXRXXX (SEQ ID NO:2).
59. The AAV capsid polypeptide of embodiment 58 comprising an amino acid sequence motif selected from the list consisting of CDCRGDCFC (SEQ ID NO:3), NDVRSAN (SEQ ID NO:4), NDVRAVS (SEQ ID NO:5), MPLGAAG (SEQ ID NO:6), NYSRGVD (SEQ ID NO:7), NEARVRE (SEQ ID NO:8), NSSRDLG (SEQ ID NO:49), SEGLKNL (SEQ ID NO:50), PSVPRPP (SEQ ID NO:51), and NFTRLSA (SEQ ID NO:52); preferably consisting of CDCRGDCFC (SEQ ID NO:3), NDVRSAN (SEQ ID NO:4), NDVRAVS (SEQ ID NO:5), MPLGAAG (SEQ ID NO:6), NYSRGVD (SEQ ID NO:7), and NEARVRE (SEQ ID NO:8).
60. The AAV capsid polypeptide of embodiments 58 or 59, wherein said amino acid sequence motif additionally comprise a small amino acid, preferably independently selected from G and A, at the N-terminus and/or at the C-terminus.
61. The AAV capsid polypeptide of any one of embodiments 58 to 60, wherein said amino acid motif additionally comprises a G at the N-terminus and an A at the at the C-terminus.
62. The AAV capsid polypeptide of any one of embodiments 58 to 61, wherein said peptide is inserted at a site of the capsid polypeptide exposed to the exterior of the AAV capsid.
33. The AAV capsid polypeptide of any one of embodiments 58 to 62, wherein said peptide is inserted at a site of the capsid polypeptide exposed to the exterior of the AAV capsid in a manner that does not interfere with the activity of said polypeptide in capsid assembly.
64. The AAV capsid polypeptide of any one of embodiments 58 to 63, wherein said peptide is inserted into the capsid polypeptide at a position after the amino acid corresponding to amino acid 587 or 588 in the AAV2 capsid polypeptide.
55. The AAV capsid polypeptide of any one of embodiments 58 to 64, wherein said peptide is inserted
   after amino acid 588 or 590 in case the capsid polypeptide is an AAV1 capsid polypeptide,
   after amino acid 587 or 588 in case the capsid polypeptide is an AAV2 capsid polypeptide,
   after amino acid 586 or 588 in case the capsid polypeptide is an AAV3 capsid polypeptide,
   after amino acid 584 or 586 in case the capsid polypeptide is an AAV4 capsid polypeptide,
   after amino acid 575 or 577 in case the capsid polypeptide is an AAV5 capsid polypeptide,
   after amino acid 588 or 590 in case the capsid polypeptide is an AAV6 capsid polypeptide,
   after amino acid 589 in case the capsid polypeptide is an AAV7 capsid polypeptide,
   after amino acid 590 in case the capsid polypeptide is an AAV8 capsid polypeptide,
   after amino acid 588 in case the capsid polypeptide is an AAV9 capsid polypeptide,
   after amino acid 590 in case the capsid polypeptide is an AAVrh. 10 capsid polypeptide,
   after amino acid 567 or 569 in case the capsid polypeptide is an AAVpo. 1 capsid polypeptide, or
   after amino acid 592 or 594 in case the capsid polypeptide is an AAV12 capsid polypeptide.
66. The AAV capsid polypeptide of any one of embodiments 58 to 65, wherein the three amino acids preceding the site into which said peptide is inserted have been changed to GQS or GQR and/or wherein the three amino acids following the site into which said peptide is inserted have been changed to QAA.
67. The AAV capsid polypeptide of any one of embodiments 58 to 66, wherein said AAV capsid polypeptide is a capsid polypeptide of an AAV serotype selected from the list consisting of AAV serotypes 1, 2, 3b, 4, 5, 6, 7, 8, 9, rh.10, po.1 and AAV12.
68. The AAV capsid polypeptide of any one of embodiments 58 to 67, wherein said AAV capsid polypeptide is a capsid polypeptide of an AAV selected from the list consisting of AAV1 (Genbank Acc No NC_002077.1, AF063497.1), AAV2 (Genbank Acc No NC_001401.2), AAV3b (Genbank Acc No AF028705.1), AAV4 (Genbank Acc No NC_001829.1, U89790.1), AAV5 (Genbank Acc No NC_006152.1), AAV6 (Genbank Acc No AF028704.1), AAV7 (Genbank Acc No NC_006260.1), AAV8 (Genbank Acc No NC_006261), AAV9 (Genbank Acc No AY530579.1), AAVrh.10 (Genbank Acc No AY243015.1), AAVpo.1 (Genbank Acc No FJ688147.1), AAV12 (Genbank Acc No DQ813647.1).
69. The AAV capsid polypeptide of any one of embodiments 58 to 68, wherein said peptide has a length of from 3 to 20 amino acids, preferably of from 4 to 12 amino acids, more preferably of from 7 to 9 amino acids.
70. A polynucleotide encoding the AAV capsid polypeptide according to any one of embodiments 58 to 69.
71. A vector comprising a polynucleotide according to embodiment 70.
72. The AAV library of any one of embodiments 1 to 5, wherein at least one of said specific combinations comprises a motif NXXR (SEQ ID NO:1), preferably NXXRXXX (SEQ ID NO:2).

All references cited in this specification are herewith incorporated by reference with respect to their entire disclosure content and the disclosure content specifically mentioned in this specification.

### Figure Legends

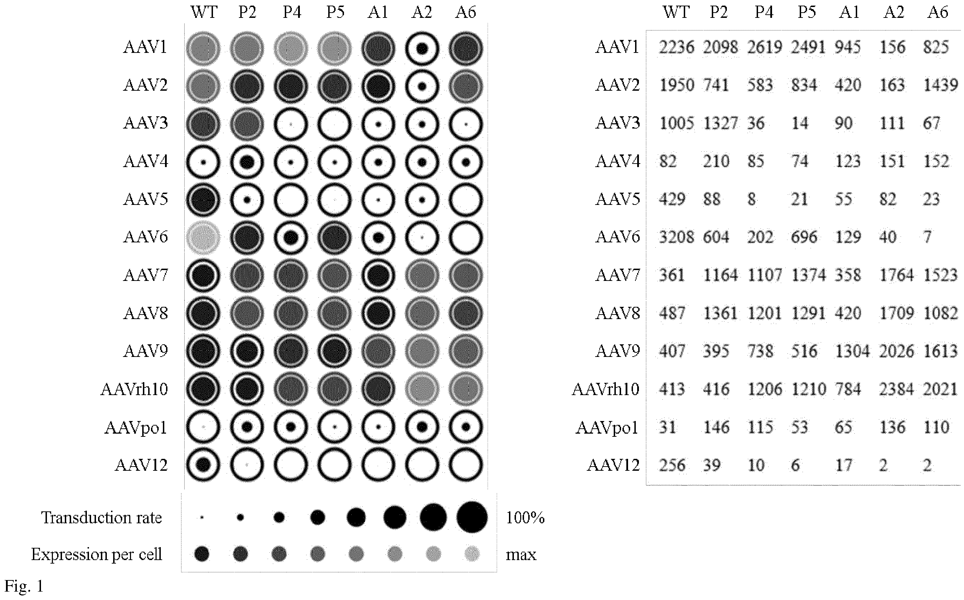

Fig. 1: Results of a screening of an AAV capsid library according to the present invention in human glioblastoma cell line U87. Transduction rates measured as percentages of YFP-expressing cells (from 0 to 100%), are indicated by the size of each bubble. Mean YFP intensities per positive cell are visualized by different shades of grey, with light grey indicating the highest intensities (1000 arbitrary units [AU]).

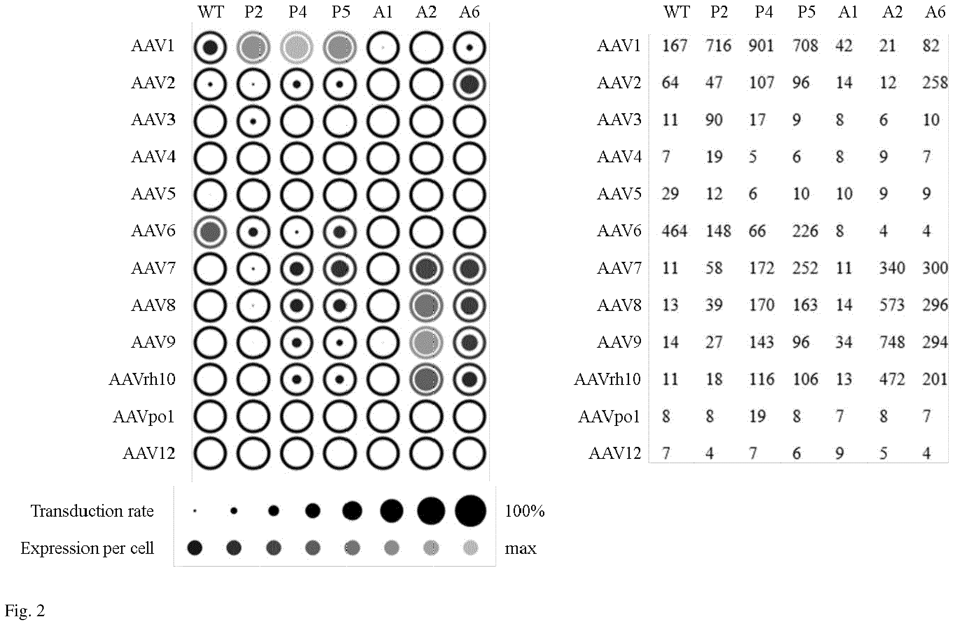

Fig. 2: Same as Fig. 1, but with human glioblastoma cell line U343. The superior performance of the peptide-modified AAV capsid mutants as compared to all wild-type AAV variants is particularly obvious in the U343 cells.

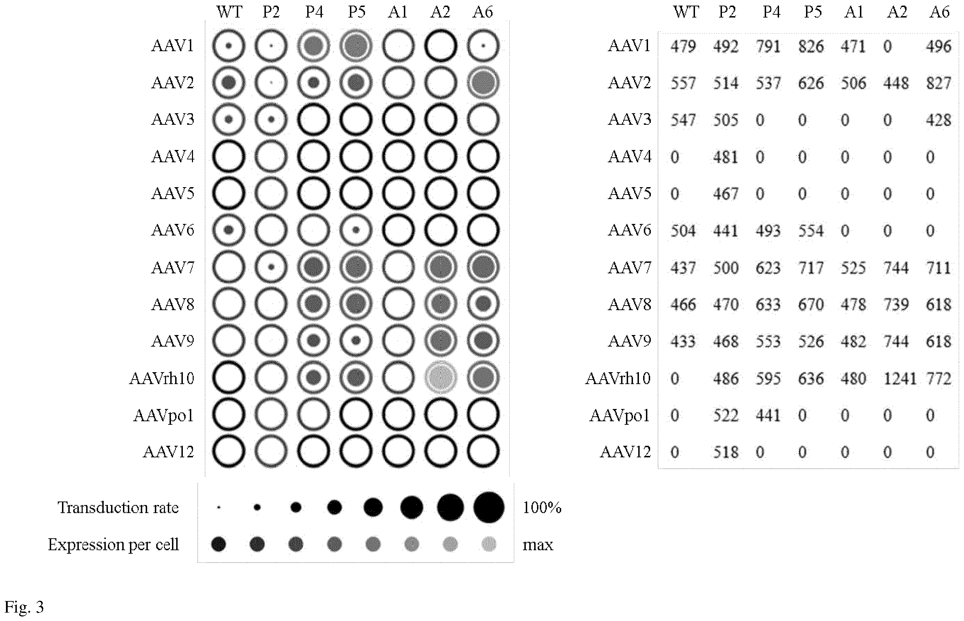

Fig. 3: Same as Fig. 1, but with hepatocellular carcinoma cell line Huh7.5.

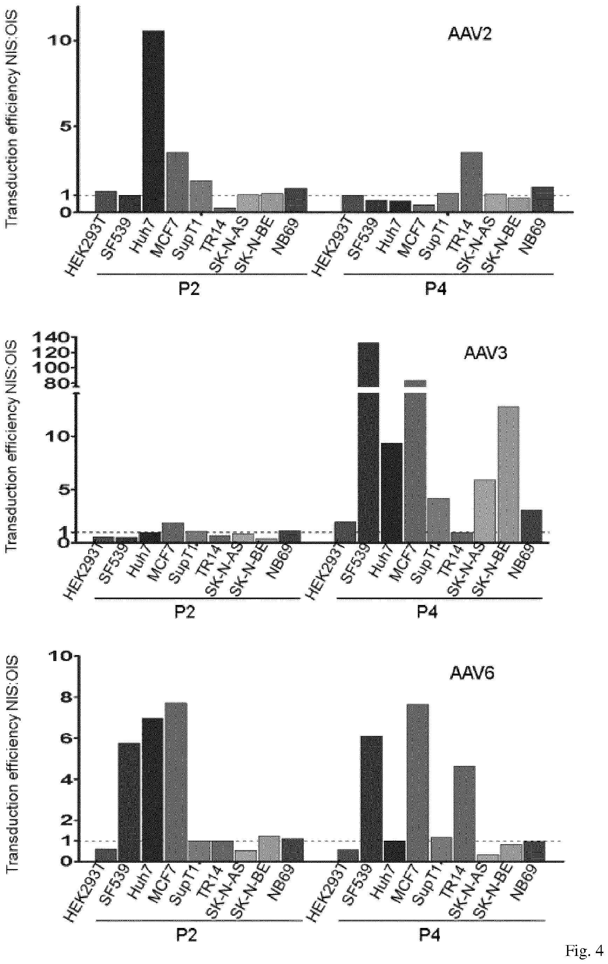

Fig. 4: Results of screening of AAV capsid libraries according to the present invention and using the altered insertions sites shown in Table 1. All vectors were prepared and tested as described in Example 1. Shown are fold-changes in transduction between the variant 2 insertion sites (NIS) and variant 1 insertion sites (OIS). P2 and P4 are inserted peptides and identical to SEQ ID No:3 or No:4, respectively. The resulting vectors were tested on the shown 9 cell lines. The dotted line indicates a NIS:OIS ratio of 1, i.e., no change. Note that use of the NIS for display of P2 but not P4 frequently improved AAV2 efficiency. The opposite was seen for AAV3, where display of P4 in the variant 2 insertion sites (NIS) enhanced the resulting particles on most of the tested cell lines, while the variant 1 insertion sites (OIS) often worked better for the P2 peptide. The AAV6 data exemplify a case where both tested peptides, P2 and P4, functioned better in the context of the NIS in several cells.

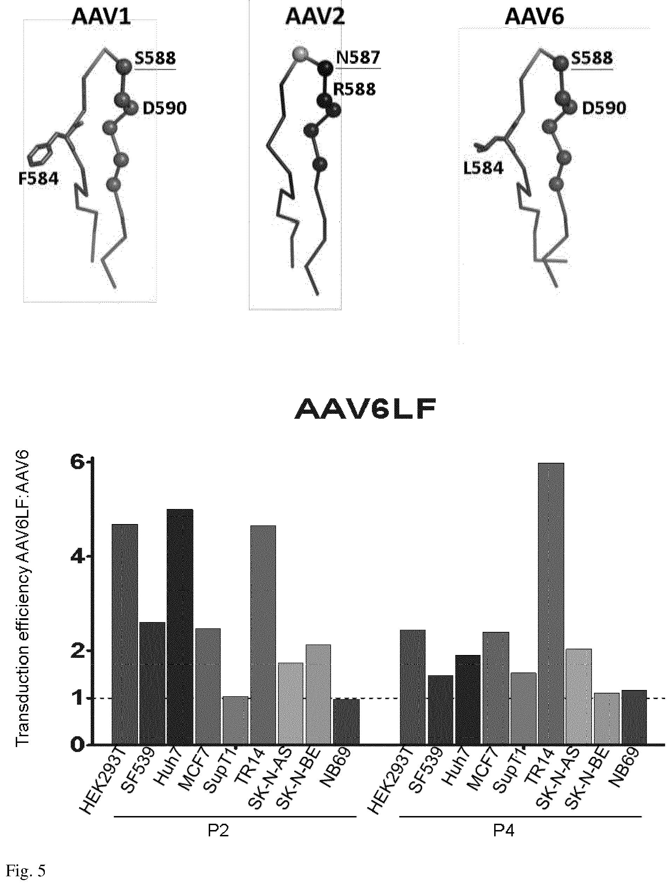

Fig. 5 Rational enhancement of peptide-displaying capsids. Shown on top is the predicted structure of the loops in the capsids of AAV1, 2 or 6 where the peptides are inserted, with the balls representing the 6 residues flanking the variant 1 insertion site (3 on each side, see also Table 1). The variant 2 insertion sites are underlined. Also highlighted is a phenylalanine in AAV1 (F584) which corresponds to a leucine in AAV6 (L584). Data at the bottom show that rational mutation of L584 in AAV6 to the F584 from AAV1 improves the efficiency of the resulting AAV particles with two different peptide insertions (P2 and P4) in most of the tested cell lines.

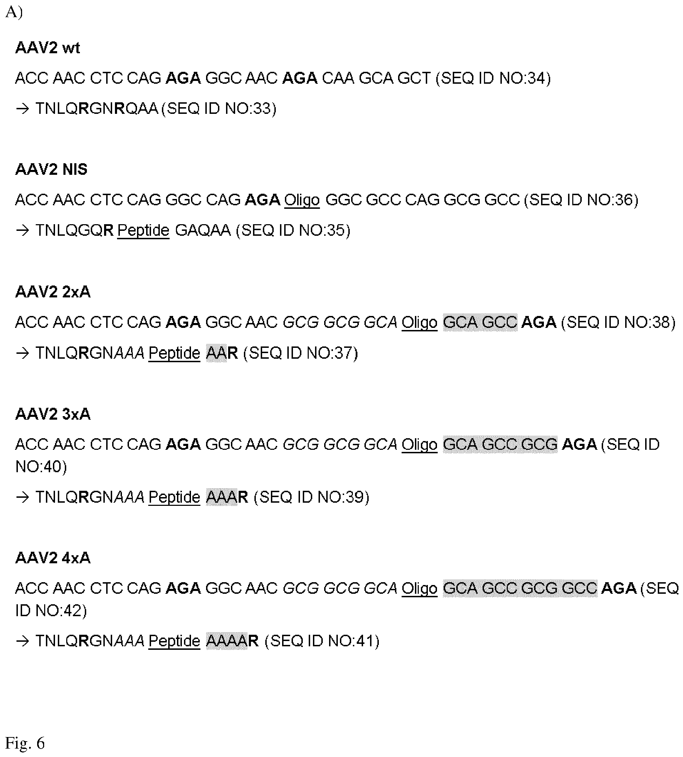

Fig. 6: Additional modification and fine-tuning of the amino acids flanking inserted peptides, using AAV2 as an example. Shown in A) are modifications that were generated and tested, differing in the number of alanines on either side of the inserted peptide, i.e., 3 on the left (italics), and 2, 3 or 4 on the right (grey shades). The combination of 3x left and 2x right corresponds to the design published by Sallach et al. in 2014 (PMID: 24468915), while the others are novel. The two arginines (R585 and R588, encoded by AGA triplets) that constitute the heparin binding motif in AAV2 are highlighted in bold. Shown in B) is a comparison of the different designs on the amino acid level, also including the variant 1 integration site (OIS). Residues that have been changed as compared to wild-type AAV2 are highlighted in medium grey. Peptide A2 was used as an example.

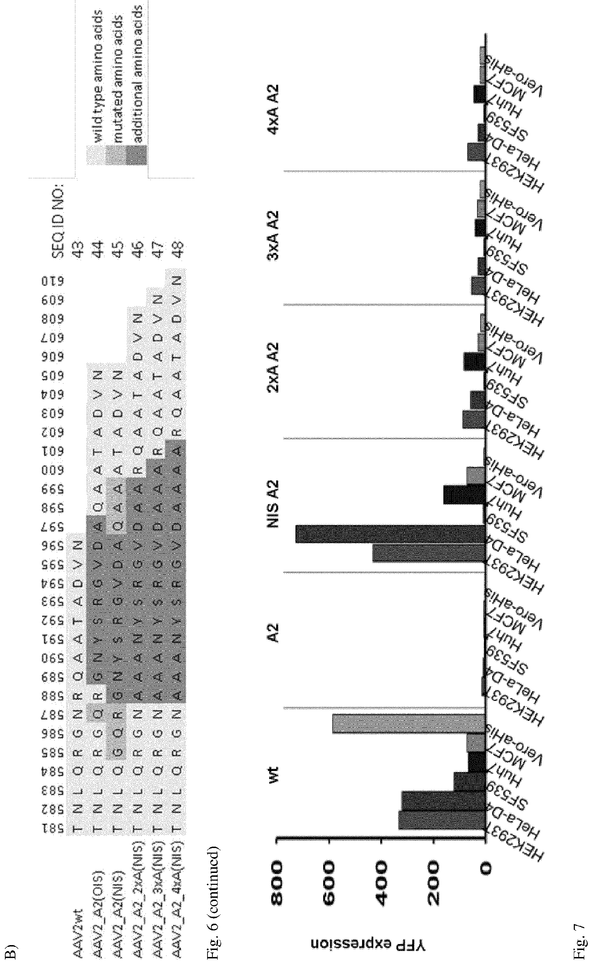

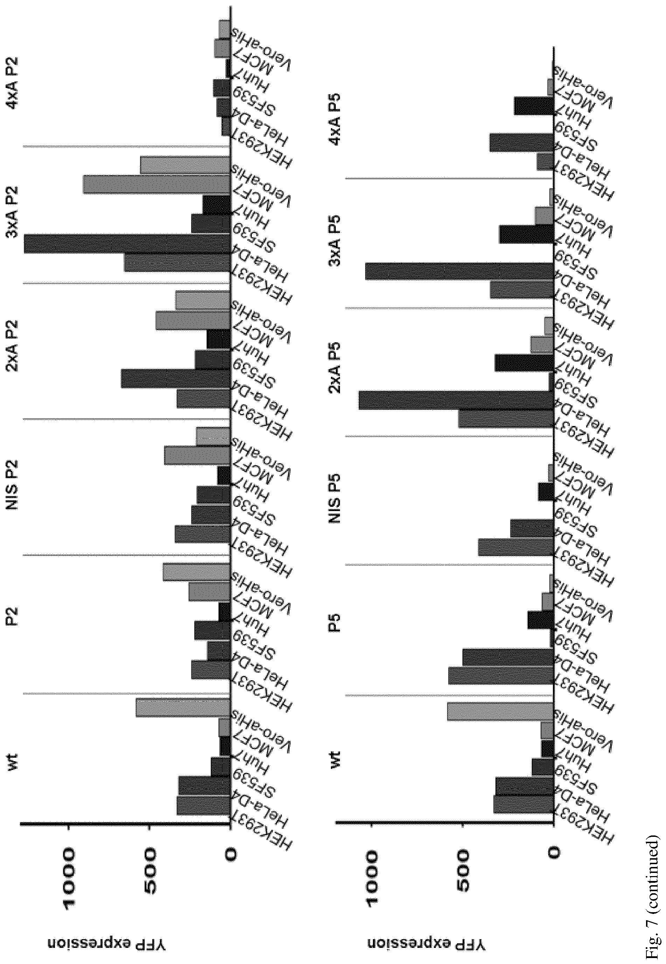

Fig. 7: Quantification of transduction of the cell lines shown with a library comprising the different capsid designs from Fig. 6 (all engineered to express a YFP reporter, used at equal amounts and quantified by microscopy). In the first example, use of the NIS gave the best results in most cells in combination with the A2 peptide (identical to SEQ ID NO:7). In the middle example (P2 peptide), the new design with the 3 alanines on the right side of the peptide gave the best results in most cell lines. Note that the data shown here represent YFP expression intensities, whereas the data with the same peptide in Fig. 4 top represent numbers of transduced cells and are thus not directly comparable. In the last panel (P5 peptide), the presence of 2 or 3 (new design) alanines on the right side of the peptide enhanced vector efficiency in most cells. wt = wild-type, i.e., not modified with a peptide.

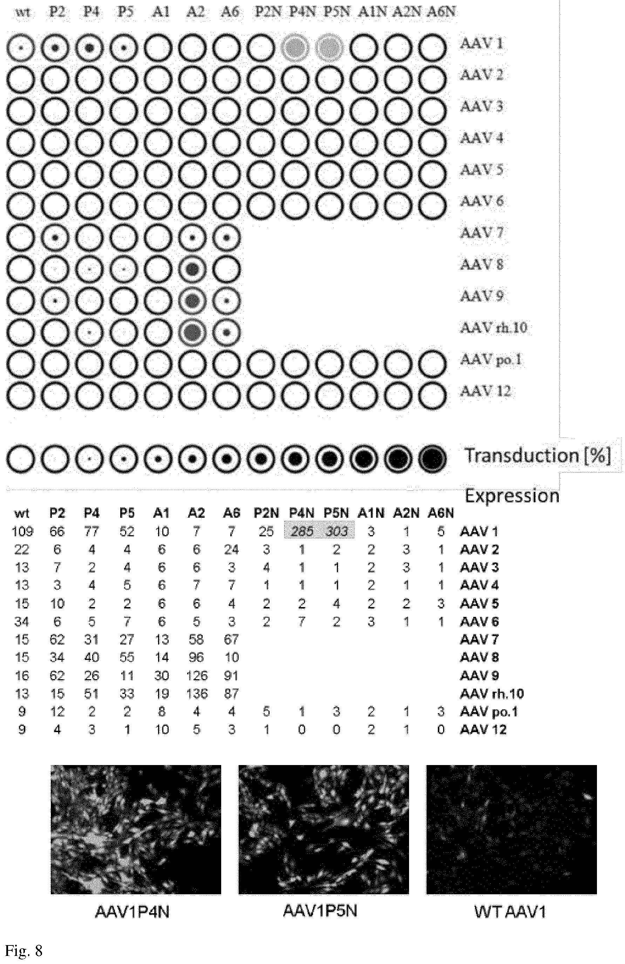

Fig. 8: Shown on top are results (transduction efficiencies, i.e., percent YFP-expressing cells) of screening of an expanded AAV capsid library comprising additional variants based on the NIS in SC-1 cells (human B-lymphocytes). In these cells, use of the variant 2 insertion site (NIS, here called N) in AAV1 in combination with the P4 and P5 peptides gave the best results. Shown underneath are corresponding YFP expression levels for each capsid within the library. The two best candidates - AAV1 with the P4 or P5 peptides in the variant 2 insertion site - are highlighted in grey. The individual results shown at the bottom further exemplify the superiority of the combination of these two peptides with the NIS in AAV1, as compared to the parental wild-type virus. For AAV7, AAV8, AAV9 and AAVrh.10, the variant 1 peptide insertion site was already in the most exposed position within the capsid; hence, there are no variant 2 derivatives.

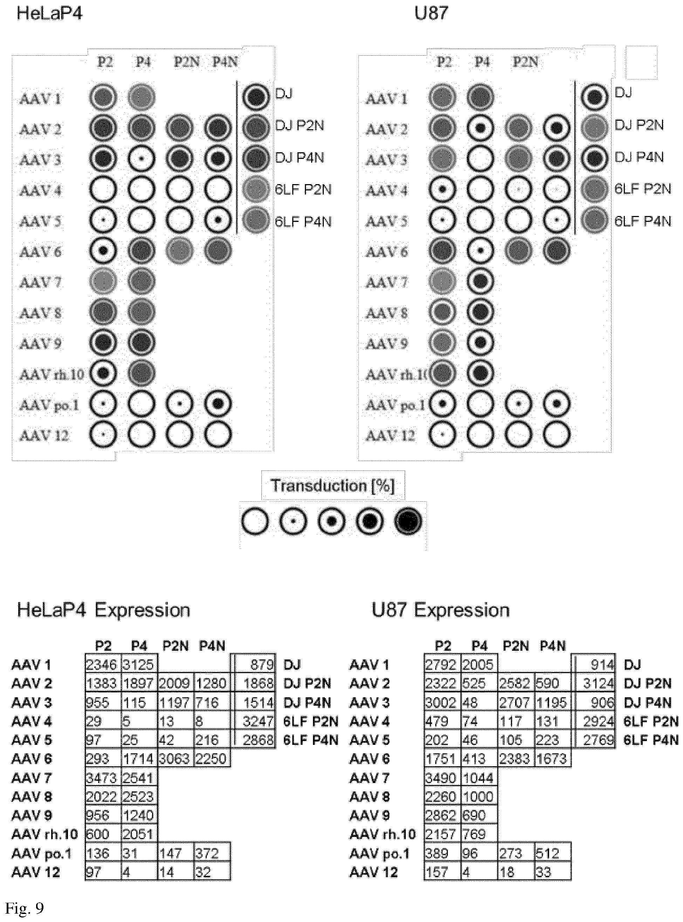

Fig. 9: Additional examples, using cell lines HeLaP4 and U87, illustrating the often beneficial effect of the shifted variant 2 insertion sites. DJ is the chimeric capsid AAV-DJ - composed of fragments from AAV2, AAV8 and AAV9 - that was created and selected by Grimm and colleagues through the molecular evolution technology of DNA family shuffling (Grimm et al. (2008), J Virol. 82(12):5887). Note how its juxtaposition with selected peptides also boosted its efficiency, demonstrating the improvements possible by combining DNA family shuffling and peptide display in the context of an AAV capsid library.

The following Examples shall merely illustrate the invention. They shall not be construed, whatsoever, to limit the scope of the invention.

### Example 1: Identifying AAV capsid variants mediating improved infectivity on specific cell lines

Two different human glioblastoma cell lines (U87 and U343) and hepatocellular carcinoma cell line Huh7.5 were used. AAV vectors were produced by transient triple-transfection of HEK293T cells grown in 6-well plates. For this, 3.5×10⁵ HEK293T cells per well were seeded in a volume of 4 mL 24 hours prior to transfection and incubated at 37°C and 5% CO2. The transfection mixture contained equal amounts of an AAV helper plasmid encoding the AAV2 rep gene together with the capsid gene coding for the respective wild-type or synthetic AAV capsid, a standard Adenovirus helper plasmid encoding all functions required for AAV vector production (E2, E4, VA), and an AAV vector construct encoding a yellow fluorescent protein (YFP) under a cytomegalovirus (CMV) promoter. The total DNA amount in the final solution was 4 µg, equivalent to 1.3 µg of each plasmid. This mixture moreover contained Dulbecco's Modified Eagle's Medium (DMEM) without supplements and TurboFect transfection reagent (8 µL per well, Thermo Scientific, Waltham, MA, USA). The entire solution was vortexed and incubated for 15 minutes at room temperature, before 400 µL of the mixture were carefully distributed per well and the cells were incubated at 37°C. After 72 hours, cells were harvested into the medium, transferred into 15 mL falcon tubes and centrifuged for 15 minutes. Following removal of the supernatant, the cell pellet was resuspended in 300 µL 1xPBS and transferred into 2 mL tubes. The samples were then subjected to five cycles of freezing and thawing (5 minutes each) using a 37°C water bath and liquid nitrogen. Finally, the samples were centrifuged for 10 minutes at 13200 rpm, and the virus particle-containing supernatant was stored at -20°C. The peptides inserted in the respective capsid polypeptides were A1: MPLGAAG (SEQ ID NO:6), A2: NYSRGVD (SEQ ID NO:7), A6: NEARVRE (SEQ ID NO:8), P2: CDCRGDCFC (SEQ ID NO:3), P4: NDVRSAN (SEQ ID NO:4), P5: NDVRAVS (SEQ ID NO:5).

To screen the entire array of vector supernatants used in Figures 1 to 3, cells were seeded at a density of 1×10⁴ cells per well in 96-well plates and one day later transduced with 5 µl of each vector stock supernatant. Forty-eight hours later, expression of the vector-encoded YFP reporter was measured via FACS analysis. Results are depicted on the left as a bubble chart. Transduction rates, i.e., percentages of YFP-expressing cells (from 0 to 100%), are indicated by the size of each bubble. Mean YFP intensities per positive cell are visualized by different shades of grey, with light grey indicating the highest intensities (measured in arbitrary units [AU]; light grey corresponds to the highest value measured within this experiment [3208, wildtype AAV6]). In addition, all AU values are shown in the table on the right. The superior performance of the peptide-modified AAV capsid mutants as compared to all wild-type AAV variants is particularly obvious in the U343 cells. Particularly obvious in U343 cells is the superior performance of several peptide-modified AAV capsid mutants, e.g., AAV1P4, AAV1P5 or AAV9A2, as compared to all wild-type AAV variants. Notably, there is a very distinct AAV transduction pattern in Huh7.5 cells (Fig. 3) as compared to the two glioblastoma cell lines (Figures 1 and 2).

### Example 2: Further data

An overview over sites in the capsids of different AAV serotypes that have been exploited to display exogenous peptides is provided in Table 1 herein above. Column "variant 2 insertion sites (NIS)" shows sites that differ from variant 1 insertion sites by 1 or 2 amino acids and that were selected based on data obtained with the original AAV capsid libraries. Columns "Modified Amino Acids" show the 3 residues that flank each peptide in the final modified capsids on the left or right side, respectively, and that differ from the cognate wild-type sequences. Numbers in #XXX (#=number, X=letter) indicate amino acid positions in each unmodified wild-type capsid, and letters represent the corresponding amino acid (in 1-letter code). For instance, P591 is a proline at position 591. Peptide insertions occurred between the two positions that are shown, e.g., D590_P591 indicates that the peptide is displayed after the aspartic acid at position 590 and before the proline at position 591.

Further data exemplifying the effects of combining wild-type (AAV2) or synthetic (AAV-DJ; Grimm et al. (2008), J Virol. 82(12):5887) capsids with different peptides and with different flanking amino acids (RR corresponds to residues R585 and R588; all other letter - QR, RS and QS - indicate single or dual point mutations) are shown in Table 2. Peptides P2, P4, P5, A2, and A6 are the same as in Example 1, the further peptides were: D1: NSSRDLG (SEQ ID NO:49), T1: SEGLKNL (SEQ ID NO:50), T2: PSVPRPP (SEQ ID NO:51), and K2: NFTRLSA (SEQ ID NO:52).

**Table 2: Transduction efficiencies of AAV variants in different cell lines (HeLa, SupT1, Jaws, and RAW); Numbers are transduction efficiencies (% YFP-positive cells).**

| **HeLa** | **2-RR** | **2-QR** | **2-RS** | **2-QS** | **DJ-RR** | **DJ-QR** | **DJ-RS** | **DJ-QS** |
|---|---|---|---|---|---|---|---|---|
| **2-RR-P2** | 90.05 | 87.76 | 89.97 | 87.39 | 51.71 | 84.22 | 60.99 | 55.9 |
| **2-RR-P4** | 97.95 | 91.38 | 94.73 | 66.75 | 98.22 | 94.99 | 91.39 | 78.97 |
| **2-RR-PS** | 1.38 | 74.91 | 91.53 | 73.17 | 91.02 | 81.64 | 92.72 | 80.23 |
| **2-RR-A2** | 1.71 | 96.99 | 2.23 | 85.6 | 96.44 | 0.11 | 91.28 | 89.26 |
| **2-RR-A6** | 1.89 | 91.71 | 1.11 | 85.64 | 94.5 | 77.41 | 85 | 77.92 |
| **2-RR-D1** | 0.58 | 96.69 | 6.21 | 77.93 | 92.25 | 90.54 | 92.56 | 94.59 |
| **2-RR-T1** | 0.04 | 13.18 | 4.7 | 3.65 | 40.06 | 15.61 | 22.62 | 16.35 |
| **2-RR-T2** | 19.22 | 39.55 | 3.09 | 28.24 | 63.61 | 30.7 | 46.79 | 32.85 |
| **2-RR-K2** | 0.05 | 76.92 | 0.03 | 86.29 | 76.27 | 68.18 | 82.8 | 70.51 |

| **SupT1** | **2-RR** | **2-QR** | **2-RS** | **2-QS** | **DJ-RR** | **DJ-QR** | **DJ-RS** | **DJ-QS** |
|---|---|---|---|---|---|---|---|---|
| **2-RR-P2** | 2.02 | 3.19 | 1.7 | 1.83 | 0.41 | 0.92 | 1.35 | 0.25 |
| **2-RR-P4** | 34.76 | 29.17 | 63.87 | 52 | 18.07 | 24.07 | 21.49 | 14.16 |
| **2-RR-PS** | 1.08 | 23.68 | 60.27 | 61.21 | 9.44 | 13.85 | 31.32 | 16.02 |
| **2-RR-A2** | 1.24 | 54.69 | 2.05 | 7.19 | 12.81 | 0 | 13.01 | 6 |
| **2-RR-A6** | 0.51 | 48.73 | 1.27 | 34.25 | 11.68 | 11.09 | 18.48 | 7.87 |
| **2-RR-D1** | 0.82 | 42.82 | 6.72 | 10.77 | 10.69 | 17.84 | 24.59 | 30.4 |
| **2-RR-T1** | 0.17 | 0.8 | 0.25 | 0.34 | 1.06 | 0.25 | 1.37 | 0.46 |
| **2-RR-T2** | 0.3 | 1.77 | 0.08 | 0.23 | 0.39 | 0.28 | 1.24 | 0 |
| **2-RR-K2** | 0.04 | 26.68 | 0.36 | 38.49 | 2.91 | 7.3 | 16.88 | 8.35 |

| **Jaws** | **2-RR** | **2-QR** | **2-RS** | **2-QS** | **DJ-RR** | **DJ-QR** | **DJ-RS** | **DJ-QS** |
|---|---|---|---|---|---|---|---|---|
| **2-RR-P2** | 0.76 | 0.56 | 0.21 | 0.71 | 1.18 | 1.38 | 0.81 | 0.54 |
| **2-RR-P4** | 3 | 2.92 | 0.83 | 0.65 | 28.61 | 18.81 | 16.34 | 6.22 |
| **2-RR-PS** | 0.73 | 0.98 | 1.37 | 0.84 | 23.04 | 12.86 | 17.05 | 6.16 |
| **2-RR-A2** | 1.14 | 0.75 | 0.3 | 0.49 | 30.78 | 0.13 | 11.5 | 4.54 |
| **2-RR-A6** | 0.88 | 0.87 | 0.62 | 0.4 | 28.12 | 14.79 | 17.22 | 5.44 |
| **2-RR-D1** | 0.68 | 0.73 | 0.38 | 0.88 | 32.48 | 22.03 | 22.3 | 8.36 |
| **2-RR-T1** | 0.4 | 0.29 | 0.3 | 0.76 | 5.13 | 2.54 | 2.63 | 1.49 |
| **2-RR-T2** | 0.89 | 0.7 | 0.67 | 0.47 | 6.06 | 3.74 | 4.62 | 3.16 |
| **2-RR-K2** | 0.35 | 0.45 | 0.39 | 1.38 | 8 | 21.17 | 21.43 | 14.66 |

| **RAW** | **2-RR** | **2-QR** | **2-RS** | **2-QS** | **DJ-RR** | **DJ-QR** | **DJ-RS** | **DJ-QS** |
|---|---|---|---|---|---|---|---|---|
| **2-RR-P2** | 0.46 | 0.12 | 0.4 | 0.03 | 5.61 | 15.78 | 10.25 | 6.15 |
| **2-RR-P4** | 5.15 | 3.85 | 1.29 | 0.13 | 50.03 | 43.91 | 27.07 | 24.71 |
| **2-RR-PS** | 0.02 | 0.72 | 3.84 | 0.28 | 41.02 | 32.99 | 50.01 | 33.7 |
| **2-RR-A2** | 0.06 | 4.24 | 0.04 | 0.41 | 62.2 | 0 | 38.47 | 33.59 |
| **2-RR-A6** | 0.06 | 5.67 | 0.07 | 1.6 | 37.11 | 21.79 | 27.04 | 21.5 |
| **2-RR-D1** | 0.04 | 2.48 | 0.04 | 0.18 | 50.49 | 46.8 | 39.09 | 49.56 |
| **2-RR-T1** | 0 | 0 | 0 | 0 | 2.11 | 1.13 | 1.42 | 0.77 |
| **2-RR-T2** | 0.01 | 0.08 | 0.03 | 0.01 | 2.79 | 1.8 | 2.54 | 1.75 |
| **2-RR-K2** | 0 | 0.53 | 0.01 | 0.79 | 21.88 | 18.54 | 32.44 | 30.08 |

## Claims

1. An AAV capsid polypeptide comprising an inserted peptide comprising an amino acid sequence motif NXXR, CDCRGDCFC (SEQ ID NO:3), MPLGAAG (SEQ ID NO:6), SEGLKNL (SEQ ID NO:50), or PSVPRPP (SEQ ID NO:51).

2. The AAV capsid polypeptide of claim 1, wherein said amino acid sequence motif is NDVRSAN (SEQ ID NO:4), NDVRAVS (SEQ ID NO:5), NYSRGVD (SEQ ID NO:7), NEARVRE (SEQ ID NO:8), NSSRDLG (SEQ ID NO:49).

3. The AAV capsid polypeptide of claim 1 or 2, wherein said amino acid motif additionally comprises a G at the N-terminus and an A at the at the C-terminus.

4. The AAV capsid polypeptide of any one of claims 1 to 3, wherein said peptide is inserted at a site of the capsid polypeptide exposed to the exterior of the AAV capsid.

5. The AAV capsid polypeptide of any one of claims 1 to 4, wherein said peptide is inserted into the capsid polypeptide at a position after the amino acid corresponding to amino acid 587 or 588 in the AAV2 capsid polypeptide.

6. The AAV capsid polypeptide of any one of claims 1 to 5, wherein said peptide has a length of from 4 to 12 amino acids, more preferably of from 7 to 9 amino acids.

7. The AAV capsid polypeptide of any one of claims 1 to 6, wherein the three amino acids preceding the site into which said peptide is inserted have been changed to GQS or GQR and/or wherein the three amino acids following the site into which said peptide is inserted have been changed to QAA.

8. The AAV capsid polypeptide of any one of claims 1 to 7, wherein said AAV capsid polypeptide is a capsid polypeptide of an AAV selected from the list consisting of AAV1 (Genbank Acc No NC_002077.1, AF063497.1), AAV2 (Genbank Acc No NC_001401.2), AAV3b (Genbank Acc No AF028705.1), AAV4 (Genbank Acc No NC_001829.1, U89790.1), AAV5 (Genbank Acc No NC_006152.1), AAV6 (Genbank Acc No AF028704.1), AAV7 (Genbank Acc No NC_006260.1), AAV8 (Genbank Acc No NC_006261), AAV9 (Genbank Acc No AY530579.1), AAVrh.10 (Genbank Acc No AY243015.1), AAVpo.1 (Genbank Acc No FJ688147.1), AAV12 (Genbank Acc No DQ813647.1), or a variant thereof.

9. The AAV capsid polypeptide of claim 8, wherein said AAV capsid polypeptide variant is a chimeric capsid polypeptide.

10. The AAV capsid polypeptide of any one of claims 1 to 9, wherein said amino acid motif is NDVRSAN (SEQ ID NO:4) and wherein said AAV capsid polypeptide is a capsid polypeptide of an AAV selected from the list consisting of AAV1, AAV2, AAV6, AAV7, AAV8, AAV9, and AAVrh.10.

11. The AAV capsid polypeptide of any one of claims 1 to 9, wherein said amino acid motif is NDVRAVS (SEQ ID NO:5) and wherein said AAV capsid polypeptide is a capsid polypeptide of an AAV selected from the list consisting of AAV1, AAV9, and AAVrh.10.

12. The AAV capsid polypeptide of any one of claims 1 to 9, wherein said amino acid motif is CDCRGDCFC (SEQ ID NO:3) and wherein said AAV capsid polypeptide is a capsid polypeptide of an AAV selected from the list consisting of AAV1, AAV2, AAV3, AAV5, AAV6, AAV7, AAV8, AAV9, AAVrh.10, AAVpo.1, and AAV12.

13. A polynucleotide encoding the AAV capsid polypeptide according to any one of claims 1 to 12.

14. A vector comprising a polynucleotide according to claim 13.

15. An AAV capsid polypeptide according to any on of claims 1 to 12, a polynucleotide according to claim 13, or a vector ccording to claim 14, for use in treating myopathy, cardiomyopathy, a neurodegenerative disorder, hemophilia type A or B, or cystic fibrosis.
